# EUROPEAN PATENT SPECIFICATION

(11) **EP 1 679 087 B1**
(45) Date of publication and mention of the grant of the patent: **20.01.2010**
(21) Application number: 05020602.8
(22) Date of filing: 21.09.2005
(51) Int. Cl.: A61L 26/00

(54) **Molecular sieve materials having increased particle size for the formation of blood clots**
Molekularsieb-Materialien mit vergrössertem Teilchendurchmesser zur Bildung von Blutgerinnseln
Matériaux contenant du tamis moléculaire ayant un diamètre de particules agrandi pour la formation de caillots sanguins

(30) Priority: 27.12.2004 US 23869
(43) Date of publication of application: 12.07.2006
(73) Proprietor: Z-Medica Corporation, Wallingford, CT 06492 (US)
(72) Inventor: Hursey, Francis X., West Hartford, Connecticut 06107 (US); Horn, Jeffrey L., Rocky Hill, Connecticut 06067 (US)
(74) Representative: Menges, Rolf

(56) References cited:
- WO-A-02/30479
- WO-A-2005/027808
- WO-A-2006/012218
- US-A- 4 822 349

## Description

The present invention relates generally to compositions for clotting blood, comprising a molecular sieve material, wherein said molecular sieve material is a zeolite in the form of particles.

Blood is a liquid tissue that includes red cells, white cells, corpuscles, and platelets dispersed in a liquid phase. The liquid phase is plasma, which includes acids, lipids, solublized electrolytes, and proteins. The proteins are suspended in the liquid phase and can be separated out of the liquid phase by any of a variety of methods such as filtration, centrifugation, electrophoresis, and immunochemical techniques. One particular protein suspended in the liquid phase is fibrinogen. When bleeding occurs, the fibrinogen reacts with water and thrombin (an enzyme) to form fibrin, which is insoluble in blood and polymerizes to form clots.

In a wide variety of circumstances, animals, including humans, can be wounded. Often bleeding is associated with such wounds. In some circumstances, the wound and the bleeding are minor, and normal blood clotting functions in addition to the application of simple first aid are all that is required. Unfortunately, however, in other circumstances substantial bleeding can occur. These situations usually require specialized equipment and materials as well as personnel trained to administer appropriate aid. If such aid is not readily available, excessive blood loss can occur. When bleeding is severe, sometimes the immediate availability of equipment and trained personnel is still insufficient to stanch the flow of blood in a timely manner.

Moreover, severe wounds can often be inflicted in remote areas or in situations, such as on a battlefield, where adequate medical assistance is not immediately available. In these instances, it is important to stop bleeding, even in less severe wounds, long enough to allow the injured person or animal to receive medical attention.

In an effort to address the above-described problems, materials have been developed for controlling excessive bleeding in situations where conventional aid is unavailable or less than optimally effective. Although these materials have been shown to be somewhat successful, they are not effective enough for traumatic wounds and tend to be expensive. Furthermore, these materials are sometimes ineffective in all situations and can be difficult to apply as well as remove from a wound.

Additionally, or alternatively, the previously developed materials can produce undesirable side effects, particularly in instances in which they are misapplied to wounds or in which they are applied by untrained personnel. For example, because prior art blood clotting material is generally a powder or in fine particulate form, the surface area of the material is relatively large. The typical moisture content of a large surface area blood clotting material is generally up to about 15% of the total weight of the material. This combination of surface area and moisture content often produces an exothermic reaction upon the application of the material to blood. Depending upon the specific surface area and the specific amount of moisture, the resulting exothermia may be sufficient to cause discomfort to or even burn the patient. Although some prior art patents specifically recite the resulting exothermia as being a desirable feature that can provide cauterization of the wound, there exists the possibility that the tissue at and around the wound site can be undesirably damaged.

WO-A-02 30479 discloses a blood coagulation accelerator that is used to promote the rate of blood clotting. The blood coagulation accelerator preferably comprises a clay material, a molecular sieve material, an inorganic oxide material, or a combination thereof.

US-A-4 822 349 discloses a method of treating wounds in which zeolite is grown from nuclei and disposed in a binder. The particlized zeolite comprises crystals that are grown from nuclei and disposed in clay binders.

Based on the foregoing, it is a general object of the present invention to provide a bleeding control material that overcomes or improves upon the prior art.

According to the present invention, a composition for clotting blood comprises a molecular sieve material wherein said molecular sieve material is a zeolite in the form of particles, wherein the particles have an average diameter of 1 mm to 7 mm or are rod-shaped and have an average length of 1 mm to 7 mm. According to an embodiment of the present invention said zeolite is an aluminosilicate including at least one of calcium and sodium. According to a further embodiment of the present invention said zeolite is an A-type crystal. Because the molecular sieve material is hydrophilic in nature, the crystalline structure adsorbs water into the interstices of the structure when left exposed in an environment having any degree of humidity.

Surprisingly, one advantage that has been discovered is that the molecular sieve material reacts less exothermically with blood as the particle size is increased. As the particle size increases, the surface area of the particles that the blood can come into contact with decreases. However, the porous nature of the material still allows water to be wicked away to cause thickening of the blood, thereby facilitating the formation of clots. Because the particle surface area exposed to the blood is reduced, a less aggressive drawing of moisture from the blood is realized, which tempers the exothermic effects experienced at the wound site.

Still another advantage of the present invention is that it is easily applied to an open wound. Particularly when the composition is in particlized form, it can be readily removed from sterilized packaging and deposited directly at the points from which blood emanates to dress the wound. Depositing the composition typically comprises pouring the particles directly on the wound.

Disclosed herein are compositions directed to the clotting of blood and the dressing of wounds. The compositions generally comprise molecular sieve materials that can minimize or stop a flow of blood by absorbing at least portions of the liquid phases of the blood, thereby promoting clotting.

As used herein, the term "zeolite" refers to a crystalline form of aluminosilicate having the ability to be dehydrated without experiencing significant changes in the crystalline structure. The zeolite may include one or more ionic species such as, for example, calcium and sodium moieties. Typically, the zeolite is a friable material that is about 90 % by weight calcium and about 10% by weight sodium. The calcium portion contains crystals that are about 0,0005 µm (5 angstroms) in size, and the sodium portion contains crystals that are about 0,0004 µm (4 angstroms) in size. The preferred molecular structure of the zeolite is an "A-type" crystal, namely, one having a cubic crystalline structure that defines round or substantially round openings.

The zeolites may be mixed with or otherwise used in conjunction with other materials having the ability to by dehydrated without significant changes in crystalline structure. Such materials include, but are not limited to, magnesium sulfate, sodium metaphosphate, calcium chloride, dextrin, combinations of the foregoing materials, and hydrates of the foregoing materials.

Zeolites for use in the disclosed applications may be naturally occurring or synthetically produced. Numerous varieties of naturally occurring zeolites are found as deposits in sedimentary environments as well as in other places. Naturally occurring zeolites that may be applicable to the compositions described herein include, but are not limited to, analcite, chabazite, heulandite, natrolite, stilbite, and thomosonite. Synthetically produced zeolites that may also find use in the compositions and methods described herein are generally produced by processes in which rare earth oxides are substituted by silicates, alumina, or alumina in combination with alkali or alkaline earth metal oxides.

The zeolite particles may be substantially spherical or irregular (e.g., balls, beads, pellets, or the like) or in the forms of chips or flakes. Substantially spherical or irregular particles, as well as chips or flakes, are about 1 mm to about 7 mm in diameter, preferably about 2 mm to about 5 mm in diameter.

Alternatively, the zeolite particles may be rod-shaped and configured to have round, irregular, or angular cross sections. In any configuration, the rods are typically produced via an extrusion process. Particles that are rod-shaped are about 1 mm to about 7 mm in length, preferably about 2 mm to about 5 mm in length.

In any embodiment (balls, beads, pellets, flakes, chips, rods), less particle surface area is available to be contacted by blood as the particle size is increased. Therefore, the rate of clotting can be controlled by varying the particle size. Surprisingly, it has been found that by maintaining particle size within the ranges provided above, such that the material comprises discrete elements, a correlative relationship between the surface area and exothermic effects when applied to blood. Furthermore, the accumulation of moisture (which also has an effect on the exothermic effects of the zeolite) can also be controlled.

Under super-humid conditions, zeolite material can be made to have a moisture content of about 21 % by weight. Preferably, the moisture content of the zeolite as utilized in the present invention is about 4% by weight to about 15% by weight, and more preferably about 5% by weight to about 12% by weight. In the preparation of zeolite material for the blood clotting composition of the present invention (i.e., formation of the material into particle form), an initial level of hydration of the zeolite may be controlled by the application of heat to the zeolite material either before or after the material is formed into particles. However, it has also surprisingly been found that as the particle size of the zeolite is increased, the moisture content has less of a correlative effect on any exothermia produced as the result of mixing the particlized zeolite in blood. Accordingly, at almost all ambient conditions the amount of moisture of the zeolite material is between about 4% by weight and about 10% by weight and moisture at this level has little effect on the efficacy of the zeolite as a blood clotting composition.

Various materials may be mixed with, associated with, or incorporated into the zeolites to maintain an antiseptic environment at the wound site or to provide functions that are supplemental to the clotting functions of the zeolites. Exemplary materials that can be used include, but are not limited to, pharmaceutically-active compositions such as antibiotics, antifungal agents, antimicrobial agents, anti-inflammatory agents, analgesics (e.g., cimetidine, chloro-pheniramine maleate, diphenhydramine hydrochloride, and promethazine hydrochloride), compounds containing silver ions, and the like. Other materials that can be incorporated to provide additional hemostatic functions include ascorbic acid, tranexamic acid, rutin, and thrombin. Botanical agents having desirable effects on the wound site may also be added.

Although this invention has been shown and described with respect to the detailed embodiments thereof, it will be understood by those of skill in the art that various changes may be made and equivalents may be substituted for elements thereof. In addition, modifications may be made to adapt a particular situation or material to the teachings of the invention. Therefore, it is intended that the invention not be limited to the particular embodiments disclosed in the above detailed description.

## Claims

1. A composition for clotting blood, said composition comprising:
a molecular sieve material, wherein said molecular sieve material is a zeolite in the form of particles, **characterized in that** said particles have an average diameter of 1 mm to 7 mm or are rod-shaped and have an average length of 1 mm to 7 mm.

2. The composition of claim 1, wherein said zeolite is an aluminosilicate wherein the aluminosilicate includes at least one of calcium and sodium.

3. The composition of claim 1 or 2, wherein said zeolite is an A-type crystal.

4. The composition of any of claims 1-3, wherein the composition includes an antibiotic, an antifungal agent, an antimicrobial agent, an anti-inflammatory agent, an analgesic, a compound containing silver ions, or a combination of any of the foregoing materials.

5. The composition of any of claims 1-4, wherein the composition includes a botanical agent.

6. The composition of any of claims 1-5, wherein a moisture content is up to 20% by weight.

7. The composition of claim 6, wherein the moisture content is 4% by weight to 15% by weight.

8. The composition of claim 7, wherein the moisture content is 5% by weight to 12% by weight.

9. The composition of any of claims 1-8, wherein said average diameter is 2 mm to 5 mm.

10. The composition of any of claims 1-8, wherein said average length is 2 mm to 5 mm.

## Patentansprüche

1. Zusammensetzung zur Blutgerinnung, wobei die Zusammensetzung umfasst: ein Molekularsiebmaterial, wobei das Molekularsiebmaterial ein Zeolith in Form von Partikeln ist, **dadurch gekennzeichnet, dass** die Partikeln einen mittleren Durchmesser von 1 mm bis 7 mm haben oder stabförmig sind und eine mittlere Länge von 1 mm bis 7 mm haben.

2. Zusammensetzung nach Anspruch 1, wobei der Zeolith ein Aluminosilikat ist, wobei das Aluminiumsilikat wenigstens Calcium oder Natrium enthält.

3. Zusammensetzung nach Anspruch 1 oder 2, wobei der Zeolith ein A-Typ-Kristall ist.

4. Zusammensetzung nach einem der Ansprüche 1-3, wobei die Zusammensetzung ein Antibiotikum, ein Fungizid, ein antimikrobielles Mittel, ein antiinflammatorisches Mittel, ein Schmerzmittel, eine Silberionen enthaltende Verbindung oder eine Kombination aus einem oder mehreren der vorgenannten Materialien umfasst.

5. Zusammensetzung nach einem der Ansprüche 1-4, wobei die Zusammensetzung ein botanisches Mittel umfasst.

6. Zusammensetzung nach einem der Ansprüche 1-5, wobei ein Feuchtigkeitsgehalt bis zu 20 Gew.-% beträgt.

7. Zusammensetzung nach Anspruch 6, wobei der Feuchtigkeitsgehalt 4 Gew.% bis 15 Gew.% beträgt.

8. Zusammensetzung nach Anspruch 7, wobei der Feuchtigkeitsgehalt 5 Gew.% bis 12 Gew.-% beträgt.

9. Zusammensetzung nach einem der Ansprüche 1-8, wobei der mittlere Durchmesser 2 mm bis 5 mm beträgt.

10. Zusammensetzung nach einem der Ansprüche 1-8, wobei die mittlere Länge 2 mm bis 5 mm beträgt.

## Revendications

1. Composition pour former des caillots de sang, ladite composition comprenant : un matériau de tamis moléculaire, où ledit matériau de tamis moléculaire est une zéolite sous la forme de particules, **caractérisée en ce que** lesdites particules ont un diamètre moyen de 1 mm à 7 mm ou sont en forme de tige et ont une longueur moyenne de 1 mm à 7 mm.

2. Composition selon la revendication 1, dans laquelle ladite zéolite est un aluminosilicate où l'aluminosilicate inclut au moins un élément parmi le calcium et le sodium.

3. Composition selon la revendication 1 ou 2, dans laquelle ladite zéolite est un cristal de type A.

4. Composition selon l'une quelconque des revendications 1 à 3, dans laquelle la composition inclut un antibiotique, un agent antifongique, un agent antimicrobien, un agent anti-inflammatoire, un analgésique, un composé contenant des ions argent ou une combinaison de l'un quelconque des matériaux précédents.

5. Composition selon l'une quelconque des revendications 1 à 4, dans laquelle la composition inclut un agent botanique.

6. Composition selon l'une quelconque des revendications 1 à 5, dans laquelle une teneur en humidité vaut jusqu'à 20 % en poids.

7. Composition selon la revendication 6, dans laquelle la teneur en humidité est de 4 % en poids à 15 % en poids.

8. Composition selon la revendication 7, dans laquelle la teneur en humidité est de 5 % en poids à 12 % en poids.

9. Composition selon l'une quelconque des revendications 1 à 8, dans laquelle ledit diamètre moyen est de 2 mm à 5 mm.

10. Composition selon l'une quelconque des revendications 1 à 8, dans laquelle ladite longueur moyenne est de 2 mm à 5 mm.
